# EUROPÄISCHE PATENTANMELDUNG

(11) **EP 2 433 659 A2**
(43) Veröffentlichungstag der Anmeldung: **28.03.2012**
(21) Anmeldenummer: 11173733.4
(22) Anmeldetag: 13.07.2011
(51) Int. Cl.: A61L 31/02, A61L 31/08

(54) **Implantat und Verfahren zur Herstellung desselben**

(30) Priorität: 13.08.2010 US 373283 P
(71) Anmelder: Biotronik AG, 8180 Bülach (CH)
(72) Erfinder: Bayer, Ullrich, 18211 Admannshagen-Bargeshagen (DE); Baader, Felix, 8193 Eglisau (CH); Wittchow, Eric, 90403 Nürnberg (DE)
(74) Vertreter: Lindner-Vogt, Karin L.

(57) **Zusammenfassung**

Die Erfindung betrifft ein Implantat, insbesondere intraluminale Endoprothese, mit einem Implantatkörper (2, 4) enthaltend eine Aluminium-Verbindung, vorzugsweise eine Aluminiumlegierung und/oder Aluminiumoxid. Eine verbesserte Biokompatibilität des Implantats wird dadurch erzielt, dass mindestens der Teil der Oberfläche des Implantatkörpers (2, 4), welche durch die Aluminium-Verbindung ausgebildet wird, eine erste Schicht (6) aufweist, welche ein Aluminiumphosphat enthält. Es wird ferner ein kostengünstiges Verfahren zur Herstellung eines derartigen Implantats beschrieben.

## Beschreibung

Die vorliegende Erfindung betrifft ein Verfahren zur Herstellung eines Implantats, insbesondere einer intraluminalen Endoprothese, mit einem Implantatkörper enthaltend eine Aluminium-Verbindung, insbesondere eine Aluminiumlegierung und/oder ein Aluminiumoxid, sowie ein entsprechendes Implantat.

Medizinische Endoprothesen oder Implantate für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents, orthopädische Implantate wie Befestigungselemente für Knochen, beispielsweise Schrauben, Platten oder Nägel, chirurgisches Nahtmaterial, Darmklammern, Gefäßclips, Prothesen im Bereich des Hart- und Weichgewebes sowie Ankerelemente für Elektroden, insbesondere von Schrittmachern oder Defibrillatoren zu verstehen.

Heute werden als Implantate besonders häufig Stents eingesetzt, die zur Behandlung von Stenosen (Gefäßverengungen) dienen. Sie weisen einen Körper in der Form eines ggf. durchbrochenen rohrförmigen oder hohlzylinderförmigen Grundgitters auf, das an beiden Längsenden offen ist. Der Implantatkörper einer derartigen Endoprothese wird in das zu behandelnde Gefäß eingesetzt und dient dazu, das Gefäß zu stützen. Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Durch den Einsatz von Stents oder anderen Implantaten kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die zu einem allmählichen Zuwachsen des Stents und im schlimmsten Fall zu einem Gefäßverschluss führen können. Ein Ansatz zur Lösung dieses Problems besteht darin, den Stent bzw. andere Implantate aus einem biodegradierbaren Werkstoff zu fertigen.

Unter Biodegradation werden hydrolytische, enzymatische und andere stoffwechselbedingte Abbauprozesse im lebenden Organismus verstanden, die vor allem durch die mit dem biodegradierbaren Werkstoff des Implantats in Kontakt gelangenden Körperflüssigkeiten verursacht werden und zu einer allmählichen Auflösung der den biodegradierbaren Werkstoff enthaltenden Strukturen des Implantats führen. Das Implantat verliert durch diesen Prozess zu einem bestimmten Zeitpunkt seine mechanische Integrität. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für den Körper biodegradierbarer Implantate geeignete Werkstoffe können beispielsweise Polymere oder Metalle enthalten. Der Körper kann dabei aus mehreren dieser Werkstoffe bestehen. Gemeinsames Merkmal dieser Werkstoffe ist ihre Biodegradierbarkeit. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren vorwiegend auf Legierungen von Magnesium, Aluminium und Eisen. Die vorliegende Erfindung bezieht sich vorzugsweise auf Implantate, deren biodegradierbarer Werkstoff des Implantatkörpers zumindest teilweise Aluminium enthält, insbesondere eine Aluminiumbasislegierung (im Folgenden kurz: Aluminiumlegierung) und/oder Aluminiumoxid.

Bei der Realisierung biodegradierbarer Implantate wird angestrebt, die Degradierbarkeit entsprechend der angestrebten Therapie bzw. der Anwendung des jeweiligen Implantats (koronar, intracranial, renal etc.) zu steuern. Für viele therapeutische Anwendungen ist beispielsweise ein wichtiger Zielkorridor, dass das Implantat in einem Zeitraum von vier Wochen bis sechs Monaten seine Integrität verliert. Hierbei wird unter Integrität, d. h. mechanischer Integrität, die Eigenschaft verstanden, dass das Implantat gegenüber dem undegradierten Implantat kaum mechanische Einbußen besitzt. Dies bedeutet, dass das Implantat mechanisch noch so stabil ist, dass zum Beispiel der Kollapsdruck lediglich geringfügig, d. h. höchstens auf 80% des Nominalwerts, abgefallen ist. Das Implantat kann somit bei vorhandener Integrität seiner Hauptfunktion, die Durchlässigkeit des Gefäßes zu gewährleisten, noch nachkommen. Alternativ kann die Integrität dadurch definiert werden, dass das Implantat mechanisch so stabil ist, dass es in seinem Belastungszustand in dem Gefäß kaum geometrischen Veränderungen unterworfen ist, beispielsweise nicht nennenswert zusammensackt, d. h. unter Belastung mindestens 80% des Dilatationsdurchmessers aufweist, oder im Fall eines Stents kaum angebrochene tragende Streben aufweist.

Implantate mit einer Eisenlegierung, insbesondere eisenhaltige Stents, sind besonders kostengünstig und einfach herstellbar. Beispielsweise für die Behandlung von Stenosen verlieren diese Implantate allerdings ihre mechanische Integrität bzw. Stützwirkung erst nach einem vergleichsweise langen Zeitraum, d.h. erst nach einer Verweildauer in dem behandelten Organismus von ca. 2 Jahren. Dies bedeutet, dass sich der Kollapsdruck bei eisenhaltigen Implantaten für die gewünschten Anwendungen über der Zeit zu langsam verringert.

Demgegenüber degradieren Implantate mit einer Magnesiumlegierung häufig zu schnell. Magnesium-Implantate lassen sich zudem schlecht verformen.

Eine weitere Möglichkeit besteht darin, Implantate überwiegend aus einer Aluminiumlegierung zu fertigen. Der Vorteil der Verwendung von Aluminiumlegierungen für Implantate besteht darin, dass diese aufgrund ihres kubisch-flächenzentrierten Kristallgitters eine im Vergleich zu den Magnesiumlegierungen mit hexagonaler Struktur deutlich bessere Verformbarkeit aufweisen. Aluminiumlegierungen besitzen im Vergleich zu Magnesiumlegierungen einen höheren Elastizitätsmodul (etwa 70 GPa gegenüber etwa 45 GPa bei einer Magnesiumlegierung). Die guten mechanischen Eigenschaften der Aluminiumlegierung sind insbesondere nützlich bei der Stentdilatation oder bei der Herstellung des Halbzeugs, welche dann kostengünstiger durchgeführt werden kann. Ein medizinisches Implantat, das aus einer Legierung mit dem Hauptbestandteil Aluminium besteht, wird in der Druckschrift DE 197 31 021 A1 erwähnt.

Kontrovers wird im Zusammenhang mit Aluminium-Implantaten jedoch die Biokompatibilität dieses Materials diskutiert, insbesondere hinsichtlich Gefäßinflammation und Nekrosewahrscheinlichkeit.

Folglich besteht die Aufgabe der vorliegenden Erfindung darin, ein kostengünstiges Verfahren zur Herstellung eines Implantats anzugeben, das eine Degradation des Implantats im gewünschten Zeitfenster bewirkt. Ferner soll das nach dem Verfahren hergestellte Implantat eine gute Biokompatibilität aufweisen. Entsprechend besteht die Aufgabe der Erfindung außerdem darin, ein derartiges Implantat zu schaffen.

Die obige Aufgabenstellung wird durch ein Implantat gelöst, bei dem mindestens der Teil der Oberfläche des Implantatkörpers, welche durch die Aluminium-Verbindung ausgebildet wird, eine erste Schicht aufweist, welche ein Aluminiumphosphat enthält

Bei der vorliegenden Erfindung umfasst der Körper des Implantats mindestens einen Teil des Implantats, vorzugsweise den Hauptteil des Implantats, welcher die mechanische Integrität des Implantats bewirkt.

Die zumindest als Monolage ausgebildete erste Schicht aus dem Aluminiumphosphat (Al-PO₄) schirmt die Endothelzellen des Körpers unmittelbar nach der Implantation des Implantats von dem metallischen Aluminium der Aluminium-Verbindung ab, so dass eine Gewebeinflammation zunächst weitestgehend unterbunden wird. Dies führt zu einer hohen Gewebeverträglichkeit, welche insbesondere in den ersten Minuten und Stunden nach der Positionierung des Implantats in der Körperumgebung Zellreaktionen verhindert.

Im weiteren Verlauf kommt es unter Körpermilieubedingungen zur Unterwanderung der dünnen Schicht aus Aluminiumphosphat, welche in einem bevorzugten Ausführungsbeispiel eine Dicke von etwa 0,5 nm bis etwa 10 nm, vorzugsweise etwa 1 nm bis etwa 5 nm aufweist. Bei der Unterwanderung der ersten Schicht werden Aluminiumhydroxide gebildet. Die Bildung von Hydroxiden bewirkt zudem eine langsame Schwächung der Implantat-Integrität durch Degradation.

Die Erfinder haben daher erkannt, dass zunächst mittels einer Aluminiumphosphat-Schicht ein Kontakt zwischen metallischem Aluminium und dem Gewebe vermieden wird. Durch die zunächst beginnende Degradation der nichtmetallischen Oberfläche kommt das angrenzende Gewebe nur langsam in Kontakt mit metallischem Aluminium. Es wird dadurch eine Nekrosebildung und eine plötzlich entstehende hohe Konzentration an freien Aluminium-Ionen vermieden.

In einer Weiterbildung der Erfindung ist auf der ersten Schicht zusätzlich eine zweite Schicht enthaltend ein sauer abbaubares Polymer, vorzugsweise PLLA und/oder PLGA, vorgesehen, welche vorzugsweise eine pharmazeutisch aktive Substanz enthält. Als sauer abbaubares Polymer wird demnach insbesondere ein Polylactid, ein Polyglycosid oder ein Copolymer aus diesen, besonders bevorzugt PLLA oder PLGA, oder ein Blend aus den genannten Polymeren eingesetzt werden. Durch die Anwesenheit der sauer abbaubaren Polymere kann die Reaktionsgeschwindigkeit der Hydroxide, welche sich bei der Unterwanderung der ersten Schicht aus dem Aluminiumphosphat bilden, variieren.

Hierbei wird unter einer "pharmazeutisch aktiven Substanz" (oder therapeutisch aktive oder wirksame Substanz) im Sinne der Erfindung wird ein pflanzlicher, tierischer oder synthetischer Wirkstoff (Medikament) oder ein Hormon verstanden, das in geeigneter Dosierung als Therapeutikum zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Umgebung des Implantats hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können beispielsweise aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe, der Taxole oder Taxane, hier vorzugsweise Paclitaxel oder Sirolimus, bestehen.

Die oben beschriebenen Hydroxide haben zudem den Vorteil, dass sie als Adjuvans wirken, wenn in der zweiten Schicht (auch als Topcoat bezeichnet) pharmazeutisch aktive Substanzen (Wirkstoffträger) eingelagert sind. Hierdurch können diese in ihrem Lösungsverhalten bzw. in ihrer Elutionsrate in Abhängigkeit von der Indikation beeinflusst werden.

Das erfindungsgemäße Implantat besitzt eine Degradationsgeschwindigkeit im Bereich von 1 bis 2 Jahren.

Eine demgegenüber erhöhte Degradationsgeschwindigkeit wird dadurch erreicht, dass die Aluminiumverbindung eine Aluminiumlegierung mit Legierungselementen aus der Gruppe enthaltend Kupfer und Silber ist. Die Legierungselemente Kupfer, Silber und Indium werden vorzugsweise an den Korngrenzen des Gefüges eingelagert und beschleunigen somit die interkristalline Korrosion. Bei Anwesenheit der angegebenen Legierungselemente sinkt somit die integrale Verweilzeit im Organismus der erfindungsgemäßen Implantate im Vergleich zu Implantaten aus einer Aluminiumlegierung ohne die bevorzugten Legierungselemente. Die genannten Legierungselemente weisen zudem zytostatische oder zytotoxische Wirkungen auf und behindern somit die Proliferation.

Die genannten Legierungselemente bilden außerdem bei Überschreiten der Löslichkeitsgrenze intermetallische Verbindungen, welche auch im Korninneren als Lokalelemente wirken und die Position des Bulkmaterials in der elektrochemischen Spannungsreihe hin zu unedleren Werten verschiebt.

Weiterhin führen die Legierungselemente Kupfer, Silber und Calcium zu einer höheren Degradationsgeschwindigkeit, da sie die Mechanismen, die zur Ausbildung der korrosionsschützenden Passivschicht aus Aluminiumoxid stören. Durch Bildung von Lokalelementen wird zudem die im Bulkmaterial fortschreitende Korrosion beschleunigt.

Ein weiterer Vorteil der Legierungselemente aus der Gruppe enthaltend Kupfer und Silber besteht darin, dass diese Legierungselemente eine Mischkristallverfestigung hervorrufen, welche zu einer Erhöhung der Streckgrenze und der Zugfestigkeit führt. Hierdurch werden die mechanischen Eigenschaften des Implantats verbessert und eine Verringerung der Dimensionen der tragenden Strukturen des Implantats (z.B. die Verringerung des Durchmessers einer Stentstrebe) ermöglicht. Hierdurch kann auch eine Materialeinsparung erreicht werden.

In einem bevorzugten Ausführungsbeispiel enthält der Körper des Implantats überwiegend Aluminium, insbesondere mehr als 80 Gew.% Aluminium, besonders bevorzugt mindestens 95 Gew.% Aluminium, insbesondere in einer Legierung. Diese Legierungen besitzen die oben angegebene Degradationsdauer.

Ferner wird die obige Aufgabenstellung gelöst durch ein Verfahren umfassend die folgenden Schritte:
a) Bereitstellen des Implantatkörpers,
b) Aufbringen einer ersten Schicht enthaltend ein Aluminiumphosphat auf mindestens den Teil der Oberfläche des Implantatkörpers, welche durch die Aluminium-Verbindung ausgebildet wird, vorzugsweise mittels Phosphorsäureanodisation.

Mittels eines derartigen Verfahrens wird auf sehr kostengünstige Weise eine Verbesserung der Biokompatibilität erreicht. Insbesondere die Behandlung des Implantatkörpers mittels Phosphorsäureanodisation zur Herstellung der Aluminiumphosphat-Schicht bewirkt die Bildung von Mikrokavitäten, die eine optimale Haftung einer weiteren Schicht ermöglicht. Die relativ großen Poren (mittlerer Durchmesser der Poren von etwa 50 nm) und die rauhe äußere Oberfläche, insbesondere im Fall der Ausbildung der Aluminium-Verbindung als Aluminiumoxid, fördern das mechanische Ineinandergreifen der Strukturen des Aluminiumoxids mit einer darüber angeordneten weiteren Schicht.

In einem weiteren Ausführungsbeispiel kann auf die erste Schicht aus Aluminiumphosphat nach Schritt b) eine zweite Schicht enthaltend ein sauer abbaubares Polymer aufgetragen werden, beispielsweise ein Polylactid, ein Polyglycosid oder ein Copolymer aus diesen, besonders bevorzugt PLLA oder PLGA, oder ein Blend aus den genannten Polymeren. Dieses Polymer kann zusätzlich eine pharmazeutisch aktive Substanz enthalten. Eine solche zweite Schicht weist die oben angegebenen Vorteile auf.

Eine derartige zweite Beschichtung wird vorzugsweise mittels Aufsprühen oder mittels Tauchen in eine verdünnte Polymerlösung hergestellt. Bei der zuletzt genannten Möglichkeit wird das Lösungsmittel anschließend thermisch ausgetrieben.

Von Vorteil ist ferner, wenn der Implantatkörper vor Schritt b) mit einem hohen Kaltverfestigungsgrad versehen und/oder vor Schritt b) eine Auslagerung bei einer Temperatur von 130°C bis 180°C über einen Zeitraum von mindestens 60 Minuten bis 48 Stunden durchgeführt wird. Der hohe Kaltverfestigungsgrad und die finale Temperbehandlung verhindern ein Anwachsen der Körner der Aluminium-Legierung durch Rekristallisation. Eine interkristalline Korrosion wird hierdurch begünstigt.

Ebenfalls für die Aufbringung einer weiteren Beschichtung vorteilhaft ist, wenn der Implantatkörper vor Schritt b) mittels einer Lauge, vorzugsweise NaOH und/oder KOH, geätzt wird. Das Ätzen wird vorzugsweise bei einer Temperatur von etwa 50°C durchgeführt. Hierdurch werden etwa 4 µm tiefe Poren erzeugt, welche einen mikroskopischen Druckknopfeffekt mit der weiteren Schicht bewirken. In einem bevorzugten Ausführungsschritt wird das Ätzen der Poren mit einem Dekapieren des Implantatkörpers in 15%iger HNO₃ abgeschlossen. Diese Behandlung spült die durch das Ätzen freigelegten Legierungselemente weg und hinterlässt eine metallisch blanke Oberfläche.

Die obige Aufgabenstellung wird ferner gelöst durch ein Implantat erhältlich durch ein oben beschriebenes erfindungsgemäßes Verfahren. Ein derartiges Implantat weist die oben im Zusammenhang mit dem erfindungsgemäßen Herstellungsverfahren angegebenen Vorteile auf.

Das erfindungsgemäße Verfahren bzw. das erfindungsgemäße Implantat wird nachfolgend anhand von Beispielen und einer einzigen Figur erläutert. Dabei bilden alle gezeigten oder beschriebenen Merkmale den Gegenstand der Erfindung, unabhängig von ihrer Zusammenfassung in den Ansprüchen oder deren Rückbezüge.

Es zeigt schematisch:

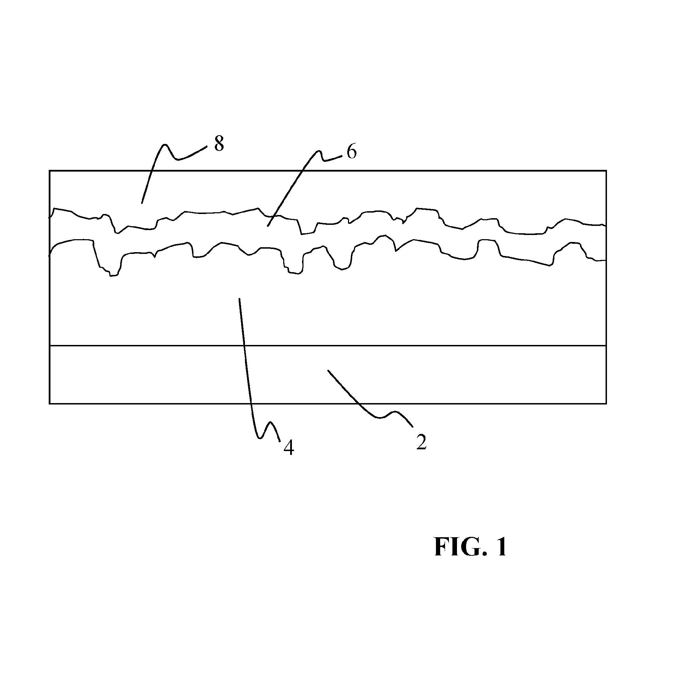

- Fig. 1: einen Querschnitt durch ein oberflächennahes Volumen eines erfindungsgemä-ßen Implantats, welches als Stent ausgebildet ist.

### 1. Beispiel:

Der Körper 2 eines Stents besteht aus einer Aluminiumlegierung mit (Angaben in Gew.%) 4% Cu, 2% Ag, 0,5% Ca. Die Rohrdimensionen des Stentkörpers betragen für den äußeren Durchmesser etwa 2 mm und die Wanddicke etwa 140 µm. Die Aluminiumlegierung weist eine Zugfestigkeit von >350 MPa, eine Streckgrenze >250 MPa bei >12% Bruchdehnung und einen Kaltverformungsgrad >30% auf. Auf der Oberfläche des Stentkörpers 2 ist häufig durch bei der Behandlung und Lagerung stattfindende Oxidationsprozesse eine etwa 2 nm bis 4 nm dicke Schicht 4 aus Aluminiumoxid gebildet.

Das Material wurde einer thermischen Auslagerung bei einer Temperatur von etwa 150°C über einen Zeitraum von etwa 1 Stunde unterzogen.

Aus einem Block oder Barren aus der angegebenen Aluminiumlegierung wird mittels bekannter Verfahren (Laserschneiden bis hin zum Elektropolieren) ein Stentkörper 2 mit der in der Beschreibungseinleitung erläuterten Gitterstruktur hergestellt. Der Stentkörper 2 wird anschließend in wässriger NaOH/KOH-Lauge (jeweils 10 Vol.% pro Komponente) bei 50°C geätzt. Danach wird der so behandelte Stentkörper über 2 Minuten zwei Mal bei 50°C in bewegtem H₂O gespült.

Der Stent wird nun in 15 Vol.% HNO₃ über einen Zeitraum von 20 Sekunden und Raumtemperatur dekapiert. Hierbei wird das Bauteil ebenfalls bewegt. Mit Ultraschallunterstützung wird anschließend zwei Mal in H₂O bei 50°C gespült. Die Oberfläche des Stentkörpers weist nach dem Ätzen und Dekapieren eine zerklüftete Struktur mit einer Reihe von Poren auf, welche eine gute Befestigung der nun erzeugten ersten Schicht 6 enthaltend die Aluminium-Verbindung ermöglicht.

Die Phosphorsäureanodisation zur Herstellung der ersten Schicht 6 enthaltend ein Aluminiumphosphat wird in 8%-iger H₃PO₄ über einen Zeitraum von 20 Minuten und bei einer Anodisierspannung von 14 V durchgeführt. Auf diesem Wege wird eine etwa 2 nm dicke AlPO₄-Schicht 6 erzeugt. Anschließend wird der beschichtete Stentkörper nochmals zwei Mal bei 50°C in H₂O gespült. Nun wird das Bauteil getrocknet.

Abschließend wird der beschichtete Stentkörper mit einem mit Wirkstoff beladenen degradierbaren Polymer, z.B. PLLA L210, mittels bekannter Verfahren besprüht. Es entsteht die zweite Schicht 8, welche eine Dicke von etwa 1 bis 2 µm aufweist. Die zweite Schicht 8 wird auch als Topcoat bezeichnet.

### 2. Beispiel:

Es wird ein Stentkörper 2 bestehend aus einer Aluminiumlegierung mit 3% Kupfer, 3% Silber, 0,2% Calzium und 0,1% Indium (Angaben jeweils in Gew.%) verwendet und einer Phosphorsäureanodisation zur Herstellung der ersten Schicht 6 enthaltend ein Aluminiumphosphat unterzogen. Hierfür wird der Stentkörper in 100%-ige H₃PO₄ eingetaucht und über einen Zeitraum von 10 Minuten und bei einer Anodisierspannung von 14 V behandelt. Auf diesem Wege wird eine etwa 2 nm dicke AlPO₄-Schicht 6 erzeugt. Anschließend wird der beschichtete Stentkörper 2 zwei Mal bei 50°C in H₂O gespült. Nun wird das beschichtete Bauteil getrocknet.

Zusätzlich können die in Beispiel 1 angegebenen Vor- und Nachbehandlungsschritte durchgeführt werden.

### 3. Beispiel:

Es wird für den Stentkörper 2 eine Legierung, wie in Beispiel 1 angegeben, verwendet und eine Phosphorsäureanodisation, wie Beispiel 2 dargestellt, durchgeführt. Der Stentkörper 2 weist dabei einen Außendurchmesser von etwa 1,8 mm und eine Wanddicke von etwa 110 µm auf.

Auch bei diesem Beispiel können die in Beispiel 1 angegebenen Vor- und Nachbehandlungsschritte durchgeführt werden.

### 4. Beispiel:

Das dritte Beispiel entspricht dem ersten Beispiel bis auf die Aufbringung des Topcoats 8. Im letzten Schritt wird ein degradierbarer, mit einem Wirkstoff (z.B. Paclitaxel) beladener Polymerblend (z.B. PLGA 85/15) derart aufgesprüht, dass eine zweite Schicht 8 mit einer Schichtdicke von ca. 1 µmbis 2 µm entsteht.

### Bezugszeichenliste

- 2: Stentkörper aus einer Aluminiumlegierung
- 4: Aluminiumoxid-Schicht, welche ebenfalls Bestandteil des Stentkörpers ist
- 6: erste Schicht
- 8: zweite Schicht

## Patentansprüche

1. Implantat, insbesondere intraluminale Endoprothese, mit einem Implantatkörper (2, 4) enthaltend eine Aluminium-Verbindung, vorzugsweise eine Aluminiumlegierung und/oder Aluminiumoxid, wobei mindestens der Teil der Oberfläche des Implantatkörpers, welche durch die Aluminium-Verbindung ausgebildet wird, eine erste Schicht (6) aufweist, welche ein Aluminiumphosphat enthält.

2. Implantat nach Anspruch 1, **dadurch gekennzeichnet, dass** die erste Schicht eine Dicke (6) von etwa 0,5 nm bis etwa 10 nm, vorzugsweise etwa 1 nm bis etwa 5 nm aufweist.

3. Implantat nach einem der vorhergehenden Ansprüche, **dadurch gekennzeichnet, dass** die Aluminiumverbindung (2, 4) eine Aluminiumlegierung mit Legierungselementen aus der Gruppe enthaltend Kupfer und Silber ist.

4. Implantat nach Anspruch 3, **dadurch gekennzeichnet, dass** die Aluminiumverbindung (2, 4) zusätzliche Legierungselemente aus der Gruppe enthaltend Calcium und Indium aufweist.

5. Implantat nach einem der vorhergehenden Ansprüche, **dadurch gekennzeichnet, dass** der Implantatkörper (2, 4) vorzugsweise überwiegend Aluminium, insbesondere mehr als 80 Gew.% Aluminium, besonders bevorzugt mindestens 95 Gew.% Aluminium enthält.

6. Implantat nach einem der vorhergehenden Ansprüche, **dadurch gekennzeichnet, dass** auf der ersten Schicht (6) zusätzlich eine zweite Schicht (8) enthaltend ein sauer abbaubares Polymer, vorzugsweise PLLA und/oder PLGA, vorgesehen ist, welche vorzugsweise eine pharmazeutisch aktive Substanz enthält.

7. Verfahren zur Herstellung eines Implantats, insbesondere einer intraluminalen Endoprothese, mit einem Implantatkörper (2, 4) enthaltend eine Aluminium-Verbindung, vorzugsweise eine Aluminiumlegierung und/oder Aluminiumoxid, umfassend die folgenden Schritte:
a) Bereitstellen des Implantatkörpers (2, 4),
b) Aufbringen einer ersten Schicht enthaltend ein Aluminiumphosphat auf mindestens den Teil der Oberfläche des Implantatkörpers (2, 4), welche durch die Aluminium-Verbindung ausgebildet wird, vorzugsweise mittels Phosphorsäureanodisation.

8. Verfahren nach Anspruch 7, **dadurch gekennzeichnet, dass** die Aluminiumverbindung eine Aluminiumlegierung mit Legierungselementen aus der Gruppe enthaltend Kupfer und Silber, und vorzugsweise weiteren Legierungselementen aus der Gruppe enthaltend Calcium und Indium ist.

9. Verfahren nach einem der Ansprüche 7 bis 8, **dadurch gekennzeichnet, dass** der Implantatkörper (2, 4) vor Schritt b) mit einem hohen Kaltverfestigungsgrad versehen und/oder eine Auslagerung bei einer Temperatur im Bereich von 130°C bis 180°C über einen Zeitraum von mindestens etwa 60 Minuten bis maximal etwa 48 Stunden durchgeführt wird.

10. Verfahren nach einem der Ansprüche 7 bis 9, **dadurch gekennzeichnet, dass** der Implantatkörper (2, 4) vor Schritt b) mittels einer Lauge, vorzugsweise NaOH und/oder KOH, geätzt wird.

11. Verfahren nach einem der Ansprüche 7 bis 10, **dadurch gekennzeichnet, dass** auf den Implantatkörper (2, 4) mit der ersten Schicht (6) zusätzlich eine zweite Schicht (8) enthaltend ein sauer abbaubares Polymer, vorzugsweise mittels Sprühen, aufgebracht wird, welche vorzugsweise eine pharmazeutisch aktive Substanz enthält.

12. Implantat, insbesondere intraluminale Endoprothese, mit einem Körper (2, 4) enthaltend eine Aluminium-Verbindung, vorzugsweise eine Aluminiumlegierung und/oder Aluminiumoxid, erhältlich durch ein Verfahren gemäß einem der Ansprüche 7 bis 11.
